# EUROPEAN PATENT APPLICATION

(11) **EP 0 827 750 A2**
(43) Date of publication of application: **11.03.1998**
(21) Application number: 97306420.7
(22) Date of filing: 22.08.1997
(51) Int. Cl.: A61K 38/22

(54) **Obesity protein formulations**

(30) Priority: 23.08.1996 US 25207 P; 23.08.1996 US 24121 P
(71) Applicant: ELI LILLY AND COMPANY, Indianapolis, Indiana 46285 (US)
(72) Inventor: Beals, John Michael, Indianapolis, Indiana 46278-1853 (US); Rinella, Joseph Vincent, Jr., Brownsburg, Indiana 46112 (US)
(74) Representative: Hudson, Christopher Mark

(57) **Abstract**

The present invention discloses a soluble parenteral formulation, comprising an obesity protein analog and an anionic amphiphilic compound.

## Description

The present invention is in the field of human medicine, particularly in the treatment of obesity and disorders associated with obesity. More specifically, the present invention relates to compounds and formulations of obesity protein.

Obesity, and especially upper body obesity, is a common and very serious public health problem in the United States and throughout the world. According to recent statistics, more than 25% of the United States population and 27% of the Canadian population are overweight. Kuczmarski, Amer. J. of Clin. Nutr. 55: 495S - 502S (1992); Reeder et. al., Can. Med. Ass. J., 23: 226-233 (1992). Upper body obesity is the strongest risk factor known for type II diabetes mellitus, and is a strong risk factor for cardiovascular disease and cancer as well. Recent estimates for the medical cost of obesity are $150,000,000,000 world wide. The problem has become serious enough that the surgeon general has begun an initiative to combat the ever increasing adiposity rampant in American society.

Much of this obesity induced pathology can be attributed to the strong association with dyslipidemia, hypertension, and insulin resistance. Many studies have demonstrated that reduction in obesity by diet and exercise reduces these risk factors dramatically. Unfortunately, these treatments are largely unsuccessful with a failure rate reaching 95%. This failure may be due to the fact that the condition is strongly associated with genetically inherited factors that contribute to increased appetite, preference for highly caloric foods, reduced physical activity, and increased lipogenic metabolism. This indicates that people inheriting these genetic traits are prone to becoming obese regardless of their efforts to combat the condition. Therefore, a pharmacological agent that can correct this adiposity handicap and allow the physician to successfully treat obese patients in spite of their genetic inheritance is needed.

The *ob /ob* mouse is a model of obesity and diabetes that is known to carry an autosomal recessive trait linked to a mutation in the sixth chromosome. Recently, Yiying Zhang and co-workers published the positional cloning of the mouse gene linked with this condition. Yiying Zhang et al. Nature 372: 425-32 (1994). This report disclosed the murine and human protein expressed in adipose tissue. Likewise, Murakami et al., in Biochemical and Biophysical Research Communications 209(3):944-52 (1995) report the cloning and expression of the rat obese gene. The protein, which is encoded by the *ob* gene, has demonstrated an ability to effectively regulate adiposity in mice. Pelleymounter et al., Science 269: 540-543 (1995).

Obesity protein analogs have been developed and have demonstrated pharmacological activity. Some of these analogs demonstrate significant improvement in physical properties and stability making them improved pharmacological agents. Analogs included in the present invention are disclosed in Basinski et al., WO 96/23515 and Basinski et al, WO 96/23517.

A parenteral formulation containing insoluble protein causes problems relating to inconsistency in the dose-response as well as unpredictability. The unpredictability is believed to be due to greater variability in the pharamacokinetics in suspension formulations. The insoluble formulations must first dissolve prior to adsorption. It is hypothesized that this step has significant varibility in a subcutaneous depot.

Unfortunately, the naturally occurring obesity proteins demonstrate a propensity to aggregate making the preparation of a soluble, pharmaceutically acceptable parenteral formulation exceedingly difficult. The molecular interactions amongst the preservative, buffer, ionic strength, pH, temperature, and any additional excipients such as a surfactant, or sugar are highly complex in view of the propensity for the obesity protein to aggregate and precipitate from the formulation. The presence of aggregate in a soluble, parenteral formulation may affect activity, bioavailability, and may result in immunological irritation and response. Indeed, irritation at the site of injection has been reported with human obesity protein.

The present invention provides conditions under which the obesity protein or an obesity protein analog is soluble and commercially viable as a multi-use pharmaceutical product. Most unexpectedly, the physical stability of the formulation is greatly enhanced in the presence of an amphiphillic compound. That is, when formulated under the conditions described herein, the obesity protein remains soluble at much higher concentrations in the presence of a phenolic preservative and at a pH range acceptable for a soluble, parenteral formulation. Furthermore, the present formulation increases temperature stability. Accordingly, the present invention provides a soluble, parenteral formulations of an obesity protein and analogs thereof.

This invention provides a soluble parenteral formulation, comprising an obesity protein and an anionic amphiphilic compound.

The invention further provides a soluble parenteral formulation of an obesity protein analog and an anionic amphiphilic compound.

The invention additionally provides the use of such formulations to treat obesity in mammals in need thereof.

For purposes of the present invention, as disclosed and claimed herein, the following terms and abbreviations are defined as follows:

Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of A with U or C with G.

C₄ to C₂₀ acylated amino acid - refers to an amino acid, preferably a naturally occurring amino acid, N-acylated with a C₄ to C₂₀, preferably a C₇ to C₂₀ acyl group. An acyl group is derived from an alkanoic acid containing four to twenty and preferably, seven to twenty carbons.

Obesity protein -- refers to the native mammalian protein produced from the native *ob* gene following transcription and deletions of introns, translation to a protein and processing to the mature protein with secretory signal peptide removed, e.g. from the N-terminal valineproline to the C-terminal cysteine of the mature protein. The human obesity protein is published in Rockefeller University WO96/05309. The rat obesity protein is published in Murakami et al., Biochemical and Biophysical Research Comm. 209(3): 944-52 (1995). Native porcine and bovine Ob proteins are disclosed in a U.S. patent application by Hansen M. Hsiung and Dennis P. Smith filed May 19, 1995, Serial number 08/445,305 (EP 0743321, published November 20, 1996). Other mammalian Ob proteins are disclosed in U.S. patent application serial number 08/452,228, filed May 26, 1995 (EP 0744408, published November 27, 1996), and provisional application, 60/003,935, filed September 19, 1995 (EP 0764722, published March 25, 1997).

Obesity protein analog -- refers to a protein of the Formula (I) : 
(SEQ ID NO: 1)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Xaa Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys (I)
 wherein:
Xaa at position 28 is Gln or absent;
said protein having at least one of the following substitutions:
Gln at position 4 is replaced with Glu;
Gln at position 7 is replaced with Glu;
Asn at position 22 is replaced with Gln or Asp;
Thr at position 27 is replaced with Ala;
Xaa at position 28 is replaced with Glu;
Gln at position 34 is replaced with Glu;
Met at position 54 is replaced with methionine sulfoxide, Leu, Ile, Val, Ala, or Gly;
Gln at position 56 is replaced with Glu;
Gln at position 62 is replaced with Glu;
Gln at position 63 is replaced with Glu;
Met at position 68 is replaced with methionine sulfoxide, Leu, Ile, Val, Ala, or Gly;
Asn at position 72 is replaced with Gln, Glu, or Asp;
Gln at position 75 is replaced with Glu;
Ser at position 77 is replaced with Ala;
Asn at position 78 is replaced with Gln or Asp;
Asn at position 82 is replaced with Gln or Asp;
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp;
Gly at position 118 is replaced with Leu;
Gln at position 130 is replaced with Glu;
Gln at position 134 is replaced with Glu;
Met at position 136 is replaced with methionine sulfoxide, Leu, Ile, Val, Ala, or Gly;
Trp at position 138 is replaced with Ala, Glu, Asp, Asn, Met, Ile, Phe, Tyr, Ser, Thr, Gly, Gln, Val or Leu; or
Gln at position 139 is replaced with Glu;
or a pharmaceutically acceptable salt thereof.

Plasmid -- an extrachromosomal self-replicating genetic element.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.

Recombinant DNA Cloning Vector -- any autonomously replicating agent including, but not limited to, plasmids and phages, comprising a DNA molecule to which one or more additional DNA. segments can or have been added.

Recombinant DNA Expression Vector -- any recombinant DNA cloning vector in which a promoter has been incorporated.

Replicon -- A DNA sequence that controls and allows for autonomous replication of a plasmid or other vector.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

Treating -- as used herein, describes the management and care of a patient for the purpose of combating the disease, condition, or disorder and includes the administration of a protein of present invention to prevent the onset of the symptoms or complications, alleviating the symptoms or complications, or eliminating the disease, condition, or disorder. Treating as used herein includes the administration of the protein for cosmetic purposes. A cosmetic purpose seeks to control the weight of a mammal to improve bodily appearance.

Isotonicity agent -- isotonicity agent refers to an agent that is physiologically tolerated and embarks a suitable tonicity to the formulation to prevent the net flow of water across the cell membrane. Compounds, such as glycerin, are commonly used for such purposes at known concentrations. Other possible isotonicity agents include salts, e.g., NaCl, dextrose, and lactose.

Physiologically tolerated buffer -- a physiologically tolerated buffer is known in the art. Physiologically tolerated buffers include TRIS, sodium acetate, sodium phosphate, or sodium citrate. The selection and concentration of buffer is known in the art.

Pharmaceutically acceptable preservative -- a multi-use parenteral formulation must meet guidelines for preservative effectiveness to be a commercially viable product. Pharmaceutically acceptable preservatives known in the art as being acceptable in parenteral formulations include: phenolic preservatives such as phenyl m-cresol, o-cresol, and/or p-cresol, benzyl alcohol, methylparaben, as well as non-phenolic preservatives chlorobutanol, phenylmercuric nitrate, thimerosal and various mixtures thereof. Other preservatives or various combinations thereof may be found, e.g., in WALLHAUSER, K.-H., DEVELOP. BIOL. STANDARD. 24, pp. 9-28 (Basel, S. Krager, 1974). The concentration necessary to achieve preservative effectiveness is dependent upon the preservative used and the conditions of the formulation.

The nucleotide and amino acid abbreviations used herein are accepted by the United States Patent and Trademark Office as set forth in 37 C.F.R. § 1.822 (b) (2) (1993). Unless otherwise indicated the amino acids are in the L configuration.

As noted above, the invention provides a soluble, multi-use parenteral formulation, comprising an obesity protein or an obesity protein analog and an anionic amphiphilic compound. Phenolics (phenol and/or cresol particularly m-cresol) or mixtures thereof are recognized as being the most preferred preservatives for multi-use parenteral formulations. However, formulations of obesity protein containing such preservatives are very unstable. In the absence of an anionic amphiphilic compound, the proteins aggregate causing the formulation to become cloudy in the presence of a phenolic. Thus, an advantage of the present invention is to provide a formulation previously believed to be unattainable. When formulated in the presence of an anionic amphiphilic compound, the protein demonstrates significantly enhanced formulation stability.

The presently claimed formulations comprise an obesity protein or analog thereof formulated with an anionic amphiphilic compound. An anionic amphiphilic compound is any compound having a water insoluble organic region (hydrophobic region) and a water soluble anionic region (hydrophilic region) and that is suitable for use in a parenteral formulation. More specifically, an anionic amphiphilic compound binds to the protein and prevents the propagation of aggregation and/or association of the protein. An anionic amphiphilic compound, therefore, includes but is not limited to,
dioctyl sodium sulfosuccinate;
a sulfate salt with-an aliphatic chain having from 4 to 20 carbons, preferably from 8 to 16 carbons, such as sodium dodecyl sulfate, sodium hexyl sulfate, or sodium octyl sulfate;
a bile salt such as sodium deoxcholate;
a salt of a fatty acid such as sodium laurate;
caprylic acid;
ascorbic acid;
ascorbyl palmitate;
an anionic phospholipids (e.g., phosphatidylserine, phosphatidylglycerol, phosphatidic acid, cardiolipin);
an N-acetylated amino acid such as N-acetyl-phenylalanine, N-acetyl-leucine, N-acetyl-L-Gly-Leu, N-acetyl-L-tryptophan;
a C₄ to C₂₀ acylated amino acid;
or a combination thereof.

A preferred anionic amphiphilic compound is dioctyl sodium sulfosuccinate. The amount of anionic amphiphilic compound in the present formulations varies with the selection of protein and the anionic amphiphilic compound employed. Generally, the amount must be sufficient to bind to the protein and prevent the propagation of aggregation and/or association of the protein. Acceptable molar ratios are from about 0.05:1 (anionic amphiphilic compound to protein) to 20:1. More preferably, 1:1 to 10:1 and most preferably, 1:1 to 5:1.

A parenteral formulation must meet guidelines for preservative effectiveness to be a commercially viable product. Preservatives known in the art as being acceptable in parenteral formulations include: phenol, m-cresol, benzyl alcohol, methylparaben, chlorobutanol, p-cresol, phenylmercuric nitrate, thimerosal and various mixtures thereof. See, e.g., WALLHAUSER, K.-H., DEVELOP. BIOL. STANDARD. 24, pp. 9-28 (Basel, S. Krager, 1974). In a preferred embodiment, the formulation contains a preservative, particularly phenol or m-cresol. The concentration of preservative required is the concentration necessary to maintain preservative effectiveness. The relative amounts of preservative necessary to maintain preservative effectiveness varies with the preservative used and any applicable pharmacopia guidelines. Generally, the amount necessary can be found in WALLHAUSER, K.-H., DEVELOP. BIOL. STANDARD. 24, pp. 9-28 (Basel, S. Krager, 1974), herein incorporated by reference. The optimal concentration of the preservative depends on the preservative, its solubility, and the pH of the formulation.

The concentration of obesity protein in the formulation is about 1.0 mg/mL to about 100 mg/mL; preferably about 5.0 mg/mL to about 50.0 mg/mL; most preferably, about 10.0 mg/mL. Parenteral daily doses of the protein are in the range from about 1 ng to about 10 mg per kg of body weight, although lower or higher dosages may be administered. The required dosage will be determined by the physician and will depend on the severity of the condition of the patient and upon such criteria as the patient's height, weight, sex, age, and medical history.

An isotonicity agent, preferably glycerin, may be additionally added to the formulation. The concentration of the isotonicity agent is in the range known in the art for parenteral formulations, preferably about 16 mg/mL. The pH of the formulation may also be buffered with a physiologically tolerated buffer, preferably a phosphate buffer, like sodium phosphate. Other acceptable physiologically tolerated buffers include TRIS, sodium acetate, or sodium citrate. The selection and concentration of buffer is known in the art; however, the formulations of the present invention are preferably prepared in the absence of salt so that the ionic strength of the formulation is minimized preferably less than 50 mM.

Other additives, such as a pharmaceutically acceptable solubilizers like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), and PEG (polyethylene glycol) or non-ionic surfactants such as polysorbate 20 or 80 or poloxamer 184 or 188, Pluronic® polyls, and other block polymers may optionally be added to the formulation to reduce aggregation. These additives are particularly useful if a pump or plastic container is used to administer the formulation. The presence of pharmaceutically acceptable surfactant mitigates the propensity for the protein to aggregate.

The parenteral formulations of the present invention can be prepared using conventional dissolution and mixing procedures. To prepare a suitable formulation, for example, a measured amount of protein in water is combined with the desired preservative in water in quantities sufficient to provide the protein and preservative at the desired concentration. The formulation is generally sterile filtered prior to administration. Variations of this process would be recognized by one of ordinary skill in the art. For example, the order the components are added, whether a surfactant is used, the temperature and pH at which the formulation is prepared may be optimized for the concentration and means of administration used.

Preferably, the pH of the present formulations is about pH 7.0 to about 9.5 and, most preferably 7.5 to 8.8. The formulations are preferably prepared under basic conditions by mixing the protein and preservative at a pH greater than pH 7.0. Preferably, the pH is about 7.5 to 8.8, and most preferably about pH 7.8. Ideally, a preservative and water solution is mixed at pH 7.5 to 8.8. Added to this solution is obesity protein in water. The pH is adjusted, as necessary, to about pH 7.5 to 8.8. The solution is then held until the components are dissolved, approximately 20 to 40 minutes, preferably about 30 minutes. The base used to adjust the pH of the formulation may be one or more pharmaceutically acceptable bases such as sodium hydroxide or potassium hydroxide. The preferred base is sodium hydroxide.

The formulations prepared in accordance with the present invention may be used in a syringe, injector, pumps or any other device recognized in the art for parenteral administration.

The preferred obesity protein analogs of the present invention are those of Formula I, wherein:
Gln at position 4 is replaced with Glu;
Gln at position 7 is replaced with Glu;
Asn at position 22 is replaced with Gln or Asp;
Thr at position 27 is replaced with Ala;
Gln at position 28 is replaced with Glu;
Gln at position 34 is replaced with Glu;
Met at position 54 is replaced with methionine sulfoxide, Leu; or Ala;
Gln at position 56 is replaced with Glu;
Gln at position 62 is replaced with Glu;
Gln at position 63 is replaced with Glu;
Met at position 68 is replaced with methionine sulfoxide, or Leu;
Asn at position 72 is replaced with Gln or Asp;
Gln at position 75 is replaced with Glu;
Asn at position 78 is replaced with Gln or Asp;
Asn at position 82 is replaced with Gln or Asp;
Gln at position 130 is replaced with Glu;
Gln at position 134 is replaced with Glu;
Met at position 136 is replaced with methionine sulfoxide, Leu, Ile; or
Gln at position 139 is replaced with Glu.

Other preferred proteins are those of Formula I wherein:
Asn at position 22 is replaced with Gln or Asp;
Thr at position 27 is replaced with Ala;
Met at position 54 is replaced with methionine sulfoxide, Leu, or Ala;
Met at position 68 is replaced with methionine sulfoxide, or Leu;
Asn at position 72 is replaced with Gln or Asp;
Asn at position 78 is replaced with Gln or Asp;
Asn at position 82 is replaced with Gln or Asp; or
Met at position 136 is replaced with methionine sulfoxide, Leu, or Ile.

Still yet additional preferred proteins are those of Formula I, wherein:
Asn at position 22 is replaced with Gln or Asp;
Thr at position 27 is replaced with Ala;
Met at position 54 is replaced with Leu, or Ala;
Met at position 68 is replaced with Leu;
Asn at position 72 is replaced with Glu or Asp;
Asn at position 78 is replaced with Gln or Asp;
Asn at position 82 is replaced with Gln or Asp; or
Met at position 136 is replaced with Leu, or Ile.

Preferred species within Formula I include species of SEQ ID NO: 2 and SEQ ID NO: 3:

(SEQ ID NO: 2)
5 10 15
Val Pro Ile Gln Lys Val Gln Asp Asp Thr Lys Thr Leu Ile Lys Thr

20 25 30
Ile Val Thr Arg Ile Asp Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85                                      90                                      95
Glu Asn Leu Arg Asp Leu Leu His Val Leu Ala Phe Ser Lys Ser Cys

                100                                     105                                     110
His Leu Pro Trp Ala Ser Gly Leu Glu Thr Leu Asp Ser Leu Gly Gly

        115                                     120                                     125
Val Leu Glu Ala Ser Gly Tyr Ser Thr Glu Val Val Ala Leu Ser Arg

    130                                     135                                     140
Leu Gln Gly Ser Leu Gln Asp Met Leu Trp Gln Leu Asp Leu Ser Pro

145
Gly Cys

(SEQ ID NO: 3)
5 10 15
Val Pro Ile Gln Lys Val Gln Asp Asp Thr Lys Thr Leu Ile Lys Thr

20 25 30
Ile Val Thr Arg Ile Asn Asp Ile Ser His Ala Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys

Most significantly, other preferred proteins of the present invention are proteins of the Formula I having at least one substitution selected from the group consisting of:
Trp at position 100 is replaced with Ala, Glu, Asp, Asn, Met, Ile, Phe, Tyr, Ser, Thr, Gly, Gln, Val or Leu; or
Trp at position 138 is replaced with Ala, Glu, Asp, Asn, Met, Ile, Phe, Tyr, Ser, Thr, Gly, Gln, Val or Leu; or a pharmaceutically acceptable salt thereof.

Other preferred proteins are a protein of the Formula II: 
(SEQ ID NO: 4)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys (II)
 said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

More preferred proteins of the Formula II are those wherein:
Trp at position 100 is replaced with Ala, Glu, Asp, Asn, Met, Ile, Phe, Tyr, Ser, Thr, Gly, Gln or Leu; or
Trp at position 138 is replaced with Ala, Glu, Asp, Asn, Met, Ile, Phe, Tyr, Ser, Thr, Gly, Gln, Val or Leu.

Yet still additional preferred proteins of the Formula II are those wherein Trp at position 100 is replaced with Ala, Glu, Asp, Gln or Leu, and most preferably Glu or Asp.

Most preferred species of the present invention are those proteins having a di-sulfide bond between Cys at position 96 and Cys at position 146. Examples of most preferred species include species of SEQ ID NO: 5-14: 
(SEQ ID NO: 5)
                    5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Ala Ala Ser Gly Leu Glu Thr Leu Asp Ser Leu Gly Gly

        115                 120                 125
 
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 6)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 7)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile
 
50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 8)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 9)
5 10 15
Val Pro Ile Gln Lys Val Gln Asp Asp Thr Lys Thr Leu Ile Lys Thr

20 25 30
Ile Val Thr Arg Ile Asn Asp Ile Ser His Ala Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 10)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly
 
115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 11)
                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Glu Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 12)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile
 
50
Leu Thr Leu Ser Lys Met Asp Gln Thr Leu Ala Val Tyr Gln Gln Ile
55 60

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Asp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or a pharmaceutically acceptable salt thereof. 
(SEQ ID NO: 13)
5 10 15
Val Pro Ile Gln Lys Val Gln Asp Asp Thr Lys Thr Leu Ile Lys Thr
20 25 30
Ile Val Thr Arg Ile Asn Asp Ile Ser His Thr Gln Ser Val Ser Ser
35 40 45
Lys Gln Lys Val Thr Gly Leu Asp Phe Ile Pro Gly Leu His Pro Ile
50 55 60
Leu Thr Leu Ser Lys Met Asp Gln Thr Leu Ala Val Tyr Gln Gln Ile
65 70 75 80
Leu Thr Ser Met Pro Ser Arg Asn Val Ile Gln Ile Ser Asn Asp Leu
85 90 95
Glu Asn Leu Arg Asp Leu Leu His Val Leu Ala Phe Ser Lys Ser Cys
100 105 110
Ser Leu Pro Gln Thr Ser Gly Leu Glu Thr Leu Asp Ser Leu Gly Gly
115 120 125
Val Leu Glu Ala Ser Gly Tyr Ser Thr Glu Val Val Ala Leu Ser Arg
130 135 140
Leu Gln Gly Ser Leu Gln Asp Met Leu Gln Gln Leu Asp Leu Ser Pro
145
Gly Cys
 said protein having a di-sulfide bond between Cys at position 96 and Cys at position 146; or a pharmaceutically acceptable salt thereof. 
(SEQ ID NO: 14)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
Ser Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or a pharmaceutically acceptable salt thereof.

The obesity proteins and obesity protein analogs of the present invention can be prepared by any of a variety of recognized peptide synthesis techniques including classical (solution) methods, solid phase methods, semi synthetic methods, and more recent recombinant DNA methods. A preferred obesity protein of the present invention is human protein of the formula: 
(SEQ ID NO: 15)
5 10 15
Xaa Val Pro Ile Gln Lys Val Gln Asp Asp Thr Lys Thr Leu Ile Lys

20 . 25 30
Thr Ile Val Thr Arg Ile Asn Asp Ile Ser His Thr Gln Ser Val Ser
 
35 40 45
Ser Lys Gln Lys Val Thr Gly Leu Asp Phe Ile Pro Gly Leu His Pro

50 55 60
Ile Leu Thr Leu Ser Lys Met Asp Gln Thr Leu Ala Val Tyr Gln Gln

65 70 75 80
Ile Leu Thr Ser Met Pro Ser Arg Asn Val Ile Gln Ile Ser Asn Asp

85 90 95
Leu Glu Asn Leu Arg Asp Leu Leu His Val Leu Ala Phe Ser Lys Ser

100 105 110
Cys His Leu Pro Trp Ala Ser Gly Leu Glu Thr Leu Asp Ser Leu Gly

115 120 125
Gly Val Leu Glu Ala Ser Gly Tyr Ser Thr Glu Val Val Ala Leu Ser

130 135 140
Arg Leu Gln Gly Ser Leu Gln Asp Met Leu Trp Gln Leu Asp Leu Ser

145
Pro Gly Cys
 wherein Xaa at position 1 is Met or absent.

The preparation of the human obesity protein is known and disclosed, for example, in Halaas Jeffrey L. et al., Science 269 (1995). The preparation of other mammalian obesity proteins are described in U.S. application serial number 08/445,305, filed May 19, 1995; U.S. patent application serial number 08/452,228, filed May 26, 1995; and provisional application, 60/003935, filed September 19, 1995; all of which are herein incorporated by reference.

The obesity protein or obesity protein analogs may be prepared by any variety of recognized peptide synthesis techniques including classical (solution) methods, solid phase methods, semi-synthetic methods, and recombinant DNA methods. The synthesis of the present obesity protein analogs is described in WO 96/23517 and WO 96/23515, herein incorporated by reference. Preferably, the obesity protein analogs prepared have one to five, and more preferably one to two substitutions selected from those identified herein.

The obesity proteins described herein may also be produced either by recombinant DNA technology or well known chemical procedures, such as solution or solid-phase peptide synthesis, or semi-synthesis in solution beginning with protein fragments coupled through conventional solution methods. Recombinant methods are preferred if a high yield is desired. The basic steps in the recombinant production of protein include:
a) construction of a synthetic or semi-synthetic (or isolation from natural sources) DNA encoding the obesity protein,
b) integrating the coding sequence into an expression vector in a manner suitable for the expression of the, protein either alone or as a fusion protein,
c) transforming an appropriate eukaryotic or prokaryotic host cell with the expression vector, and
d) recovering and purifying the recombinantly produced protein.

Synthetic genes, the in vitro or in vivo transcription and translation of which will result in the production of the protein may be constructed by techniques well known in the art. Owing to the natural degeneracy of the genetic code, the skilled artisan will recognize that a sizable yet definite number of DNA sequences may be constructed which encode the desired proteins. In the preferred practice of the invention, synthesis is achieved by recombinant DNA technology.

Methodology of synthetic gene construction is well known in the art. For example, see Brown, et al.(1979) Methods in Enzymology, Academic Press, N.Y., Vol. 68, pgs. 109-151. The DNA sequence corresponding to the synthetic claimed protein gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404). It may be desirable in some applications to modify the coding sequence of the obesity protein so as to incorporate a convenient protease sensitive cleavage site, e.g., between the signal peptide and the structural protein facilitating the controlled excision of the signal peptide from the fusion protein construct.

The gene encoding the obesity protein may also be created by using polymerase chain reaction (PCR). The template can be a cDNA library (commercially available from CLONETECH or STRATAGENE) or mRNA isolated from the desired arrival adipose tissue. Such methodologies are well known in the art Maniatis, et al. Molecular Cloning: A Laboratory Manual, Cold Spring Harbor Press, Cold Spring Harbor Laboratory, Cold Spring Harbor, New York (1989).

The constructed or isolated DNA sequences are useful for expressing the obesity protein either by direct expression or as fusion protein. When the sequences are used in a fusion gene, the resulting product will require enzymatic or chemical cleavage. A variety of peptidases which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See U.S. Patent No. 5,126,249; Carter P., Site Specific Proteolysis of Fusion Proteins, Ch. 13 in Protein Purification: From Molecular Mechanisms to Large Scale Processes, American Chemical Soc., Washington, D.C. (1990).

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. A synthetic coding sequence may be designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The restriction sites are chosen so as to properly orient the coding sequence with control sequences to achieve proper in-frame reading and expression of the protein.

In general, plasmid vectors containing promoters and control sequences which are derived from species compatible with the host cell are used with these hosts. The vector ordinarily carries a replication origin as well as marker sequences which are capable of providing phenotypic selection in transformed cells. For example, E. coli is typically transformed using pBR322, a plasmid derived from an E. coli species (Bolivar, et al., Gene 2:95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,473, the teachings of which are herein incorporated by reference. The gene encoding A-C-B proinsulin described in U.S. patent No. 5,304,473 can be removed from the plasmid pRB182 with restriction enzymes NdeI and BamHI. The isolated DNA sequences can be inserted into the plasmid backbone on a NdeI/BamHI restriction fragment cassette.

In general, procaryotes are used for cloning of DNA sequences in constructing the vectors useful in the invention. For example, E. coli K12 strain 294 (ATCC No. 31446) is particularly useful. Other microbial strains which may be used include E. coliB and E. coli X1776 (ATCC No. 31537). These examples are illustrative rather than limiting.

Procaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the β-lactamase (vector pGX2907 [ATCC 39344] contains the replicon and β-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

The DNA molecules may also be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. β-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBb (ATCC 77177). Of course, promoters from the host cell or related species also are useful herein.

Transcription of the DNA by higher eucaryotes is increased by inserting an enhancer sequence into the vector. Enhancers are cis-acting elements of DNA, usually about 10-300 bp, that act on a promoter to increase its transcription. Enhancers are relatively oriented and positioned independently and have been found 5' (Laimins, L. et al., PNAS 78:993(1981)) and 3' (Lusky, M. L., et al., Mol. Cell Bio. 3:1108 (1983)) to the transcription unit, within an intron (Banerji, J. L. et al., Cell 33:729 (1983)) as well as within the coding sequence itself (Osborne, T. F., et al., Mol. Cell Bio. 4:1293 (1984)). Many enhancer sequences are now known from mammalian genes (globin, PSV, SV40, EMC, elastase, albumin, alpha-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422 (1980), or hygromycin, Sugden, B. et al.,Mol Cell. Biol.5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH10B (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratorv Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eucaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); Chinese hamster ovary cells CHO-DHFR⁻ (ATCC CRL-9096), mouse Sertoli cells (TM4, ATCC CRL-1715, Biol. Reprod. 23:243-250(1980)); African green monkey kidney cells (VERO 76, ATCC CRL-1587); human cervical epitheloid carcinoma cells (HeLa, ATCC CCL-2); canine kidney cells (MDCK, ATCC CCL-34) ; buffalo rat liver cells (BRL 3A, ATCC CRL-1442) ; human diploid lung cells (WI-38, ATCC CCL-75); human hepatocellular carcinoma cells (Hep G2, ATCC HB-8065);and mouse mammary tumor cells (MMT 060562, ATCC CCL51).

In addition to prokaryotes, unicellular eukaryotes such as yeast cultures may also be used. Saccharomvces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10 :157(1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12(1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which contain inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRyl21 ATCC 37658) utilization. Suitable vectors and promoters for use in yeast expression are further described in R. Hitzeman et al., European Patent Publication No. 73,657A. Yeast enhancers such as the UAS Gal from Saccharomvces cerevisiae (found in conjunction with the CYC1 promoter on plasmid YEpsec--hIlbeta ATCC 67024), also are advantageously used with yeast promoters.

### Preparation Porcine OB Gene and Gene Product

Total RNA was isolated from porcine fat tissue obtained from Pel-Freez®, Pel-Freez Inc., and the cDNA was cloned in accordance with the techniques described in Hsiung et al., Neuropeptide 25: 1-10 (1994).

Primers were designed based on the published amino acid sequence of the human *ob* gene. The primers were prepared for use in polymerase chain reaction (PCR) amplification methods using a Model 380A DNA synthesizers (PE-Applied Biosystems, Inc., 850 Lincoln Center Drive, Foster City, CA 94404). Primers PCROB-1 (12504) (ATG CAT TGG GGA MCC CTG TG) (SEQ ID NO: 16), PCROB-2 (12505) (GG ATT CTT GTG GCT TTG GYC CTA TCT) (SEQ ID NO: 17), PCROB-3 (12506) (TCA GCA CCC AGG GCT GAG GTC CA) (SEQ ID NO: 18), and PCROB-4 (12507) (CAT GTC CTG CAG AGA CCC CTG CAG CCT GCT CA)(SEQ ID NO: 19) were prepared.

For cDNA synthesis, total RNA 1 µL (1 µg/µl) isolated from porcine adipose tissue and 1 µL Perkin Elmer Random primers (50 µM) in a total volume of 12 µL were annealed for 10 minutes at 70°C and then cooled on ice. The following were then added to the annealed mixture: 4 µL of BRL 5x H-reverse transcriptase (RT) reaction buffer (Gibco-BRL CAT#28025-013), 2 µL of 0.1 M DTT, 1 µL of 10 mM dNTPs. This annealed mixture was then incubated at 37°C for 2 minutes before adding 1 µL BRL M-MLV-reverse transcriptase (200 U/µL) (CAT#28025-013) and incubated at 37°C for additional 1 hour. After incubation the mixture was heated at 95°C for 5 minutes and then was cooled on ice.

For amplification of cDNA, the polymerase chain reaction (PCR) was carried out in a reaction mixture (100 µL) containing the above cDNA reaction mixture (1 µL), 2.5 units of AmpliTaq DNA polymerase (Perkin-Elmer Corporation) 10 µl of 10x PCR reaction buffer (Perkin-Elmer Corporation) and 50 pmol each of the sense (PCROB-1) and antisense (PCROB-3) primers for porcine OB amplification. The condition for PCR was 95°C for 1 minute, 57°C for 1 minute and 72⁻°C for 1 minute for 30 cycles using a PCR DNA Thermal Cycler (Perkin-Elmer Corporation). After PCR amplification, 5 µL BRL T4 DNA polymerase (5 U/µL), 2 µL BRL T4 polynucleotide kinase (10 U/µL), and 5 µL ATP (10 mM) were added to the PCR reaction mixture (100 µl) directly and incubated for 30 minutes at 37°C. After the incubation the reaction mixture was heated at 95°C for 5 minutes and then was cooled on ice. The 500 bp fragment (~0.5 mg) was purified by agarose gel electrophoresis and isolated by the freeze-squeeze method. The 500 bp fragment (~0.2 µg) was then ligated into SmaI linearized pUC18 plasmid (~1 µg) and the ligation mixture was used to transform DH5a (BRL) competent cells. The transformation mixture was plated on 0.02% X-Gal TY broth plates containing ampicillin (Amp) (100 µg/mL) and was then incubated overnight at 37°C. White clones were picked and were grown at 37°C overnight in TY broth containing Amp (100 µg/mL). The plasmid was isolated using a Wizard Miniprep DNA purification system (Promega) and submitted for DNA sequencing on a Applied Biosystem 370 DNA sequencer.

### Preparation 2

### Bovine OB Gene and Gene Product

The DNA sequence of the bovine OB gene was obtained by techniques analogous to Example 1, except sense (PCROB-2) and antisense (PCROB-3) primers were used for bovine OB cDNA amplification.

### Preparation 3

### Vector Construction

A plasmid containing the DNA sequence encoding the obesity protein is constructed to include NdeI and BamHI restriction sites. The plasmid carrying the cloned PCR product is digested with NdeI and BamHI restriction enzymes. The small ~ 450bp fragment is gel-purified and ligated into the vector pRB182 from which the coding sequence for A-C-B proinsulin is deleted. The ligation products are transformed into E. coli DH10B (commercially available from GIBCO-BRL) and colonies growing on tryptone-yeast (DIFCO) plates supplemented with 10 mg/mL of tetracycline are analyzed. Plasmid DNA is isolated, digested with NdeI and BamHI and the resulting fragments are separated by agarose gel electrophoresis. Plasmids containing the expected ~ 450bp NdeI to BamHI fragment are kept. E. coli K12 RV308 (available from the NRRL under deposit number B-15624) are transformed with this second plasmid, resulting in a culture suitable for expressing the protein.

The techniques of transforming cells with the aforementioned vectors are well known in the art and may be found in such general references as Maniatis, et al. (1988) Molecular Cloning: A Laboratory Manual, Cold Spring Harbor Press, Cold Spring Harbor Laboratory, Cold Spring Harbor, New York or Current Protocols in Molecular Biology (1989) and supplements. The techniques involved in the transformation of E. coli cells used in the preferred practice of the invention as exemplified herein are well known in the art. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift from about 30 to about 40 degrees C. in the culture conditions so as to induce protein synthesis.

In the preferred embodiment of the invention, E. coli K12 RV308 cells are employed as host cells but numerous other cell lines are available such as, but not limited to, E. coli K12 L201, L687, L693, L507, L640, L641, L695, L814 (E. coli B). The transformed host cells are then plated on appropriate media under the selective pressure of the antibiotic corresponding to the resistance gene present on the expression plasmid. The cultures are then incubated for a time and temperature appropriate to the host cell line employed.

Proteins that are expressed in high-level bacterial expression systems characteristically aggregate in granules or inclusion bodies which contain high levels of the overexpressed protein. Kreuger et al., in Protein Folding, Gierasch and King, eds., pgs 136-142 (1990), American Association for the Advancement of Science Publication No. 89-18S, Washington, D.C. Such protein aggregates must be solubilized to provide further purification and isolation of the desired protein product. Id. A variety of techniques using strongly denaturing solutions such as guanidinium-HCl and/or weakly denaturing solutions such as dithiothreitol (DTT) are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

Preferably, the DNA sequences are expressed with a dipeptide leader sequence encoding Met-Arg or Met-Tyr as described in U.S. Patent No. 5,126,249, herein incorporated by reference. This approach facilitates the efficient expression of proteins and enables rapid conversion to the active protein form with Cathepsin C or other dipeptidylpeptidases. The purification of proteins is by techniques known in the art and includes reverse phase chromatography, affinity chromatography, and size exclusion.

The influence of anionic amphiphilic compounds on the physical stability of obesity protein or analogs thereof is studied using a turbidimetric assay to kinetically monitor aggregation under formulation conditions. The data are analyzed using a linear regression procedure.

The addition of various anionic amphiphilic molecules with anionic hydrophilic groups to formulation of the obesity protein or the protein analogs defined herein produced affects in either the rate of aggregation, the extent of initial aggregation, or both. The anionic amphiphilic compounds reduce the extent of aggregation (y-intercept) at 37°C relative to the control. The order of effectiveness is believed to be dioctyl sodium sulfosuccinate > sodium dodecyl sulfate > sodium laurate > sodium deoxycholate. In addition, dioctyl sodium sulfosuccinates sodium dodecyl sulfate, and sodium laurate produce measurable reductions in the rate of aggregation at 25°C, relative to the control. The most preferred additive is dioctyl sodium sulfosuccinate which is most effect at both reducing the rate of aggregation and the extent of initial aggregation at all temperatures.

The addition of anionic amphiphilic molecules with anionic hydrophilic groups improves the physical stability of the protein and reduces the temperature sensitive of these formulations. These advances improve the robustness of the obesity protein formulation with respect to physical stability and most notable in the presence of phenolic preservatives that can have detrimental effect on the obesity protein solubility. Consequently, this invention can provide a solution formulation with the necessary physical stability and preservative effectiveness to allow a more flexible patient usage pattern, increased compliance, and improved anti-microbial protection.

The following examples are provided merely to further illustrate the preparation of the formulations of the invention. The scope of the invention is not construed as merely consisting of the following examples.

### Example 1

### Preparation of M-Cresol/Dioctyl Sodium

### Sulfosuccinate Formulation

To generate a formulation containing a protein of SEQ ID NO: 15 wherein Xaa is Met (hereafter, Protein NO: 15) the lyophilized solid bulk material is first dissolved in water containing a buffer, e.g., arginine (11 mM), at alkaline pH conditions (stock 1). The concentration of Protein NO: 15 (22 mg/mL) is verified by UV/VIS spectrophotometry using the known extinction coefficient for Protein NO: 15 at the maximum spectral absorbance (279 nm or 280 nm), measuring the maximum spectral absorbance (279 nm or 280 nm), and utilizing the dilution factor. Stock solutions containing the anionic amphiphilic compound, dioctyl sodium sulfosuccinate (0.56%), and the preservative, m-cresol (92.3 mM), are prepared by dissolving the solid in water (stocks 2 and 3, respectively). A formulation containing Protein NO: 15 at 10 mg/mL is prepared by addition of an aliquot of stock 1 (4.5 mL) to a 10 mL volumetric flask. Aliquots of stocks 2 (1.0 mL), stock 3 (3.0 mL), and water are added in order. Enough water is added to adjust the final volume to 10 mL. The pH of the solution is checked and, if necessary, the pH is adjusted with µL quantities of dilute HCl or dilute NaOH to a pH 8.5 (± 0.1). The final solution condition is 10 mg/mL of Protein NO: 6, 27.6 mM m-cresol, and 0.056% dioctyl sodium sulfosuccinate at pH 8.5 (± 0.1). The solution is then distributed into vials and stored at the desired temperatures.

### Example 2

### Preparation of M-Cresol/Sodium Dodecyl Sulfate Formulation

To generate a formulation containing a protein of SEQ ID NO: 15 (wherein Xaa is absent, hereafter, Protein NO: 15) the lyophilized solid bulk material is first dissolved in water containing a buffer, e.g., arginine (11 mM), at alkaline pH conditions (stock 1). The concentration of Protein NO: 15 (22 mg/mL) is verified by UV/VIS spectrophotometry using the known extinction coefficient for Protein NO: 1 at the maximum spectral absorbance (279 nm or 280 nm), measuring the maximum spectral absorbance (279 nm or 280 nm), and utilizing the dilution factor. Stock solutions containing the anionic amphiphilic compound, sodium dodecyl sulfate (0.36%), and the preservative, m-cresol (92.3 mM), are prepared by dissolving the solid in water (stocks 2 and 3, respectively). A formulation containing Protein NO: 15 at 10 mg/mL is prepared by addition of an aliquot of stock 1 (4.5 mL) to a 10 mL volumetric flask. Aliquots of stocks 2 (1.0 mL), stock 3 (3.0 mL), and water are added in order. Enough water is added to adjust the final volume to 10 mL. The pH of the solution is checked and, if necessary, the pH is adjusted with µL quantities of dilute HCl or dilute NaOH to a pH 8.5 (± 0.1). The final solution condition is 10 mg/mL of Protein NO: 15, 27.6 mM m-cresol, and 0.036% sodium dodecyl sulfate at pH 8.5 (± 0.1). The solution is then distributed into vials and stored at the desired temperatures.

### Example 3

### Preparation of M-Cresol/Dioctyl Sodium Sulfosuccinate Formulation

To generate a formulation containing a protein of SEQ ID NO: 5 (hereafter, Protein NO: 5) the lyophilized solid bulk material was first dissolved in water containing a buffer, e.g., arginine (11 mM), at alkaline pH conditions (stock 1). The concentration of Protein NO: 5 (22 mg/mL) was verified by UV/VIS spectrophotometry using the known extinction coefficient for Protein NO: 5 at the maximum spectral absorbance (279 nm or 280 nm), measuring the maximum spectral absorbance (279 nm or 280 nm), and utilizing the dilution factor. Stock solutions containing the anionic amphiphilic compound, dioctyl sodium sulfosuccinate (0.56%), and the preservative, m-cresol (92.3 mM), were prepared by dissolving the solid in water (stocks 2 and 3, respectively). A formulation containing Protein NO: 6 at 10 mg/mL was prepared by addition of an aliquot of stock 1 (4.5 mL) to a 10 mL volumetric flask. Aliquots of stocks 2 (1.0 mL), stock 3 (3.0 mL), and water were added in order. Enough water was added to adjust the final volume to 10 mL. The pH of the solution was checked and, if necessary, the pH was adjusted with µL quantities of dilute HCl or dilute NaOH to a pH 8.5 (± 0.1). The final solution condition was 10 mg/mL of Protein NO: 5, 27.6 mM m-cresol, and 0.056% dioctyl sodium sulfosuccinate at pH 8.5 (± 0.1). The solution was then distributed into vials and stored at the desired temperatures.

### Example 4

### Preparation of M-Cresol/Sodium Dodecyl Sulfate Formulation

To generate a formulation containing a protein of Seq ID NO: 5 (hereafter, Protein NO: 5) the lyophilized solid bulk material was first dissolved in water containing a buffer, e.g., arginine (11 mM), at alkaline pH conditions (stock 1). The concentration of Protein NO: 5 (22 mg/mL) was verified by W/VIS spectrophotometry using the known extinction coefficient for Protein NO: 5 at the maximum spectral absorbance (279 nm or 280 nm), measuring the maximum spectral absorbance (279 nm or 280 nm), and utilizing the dilution factor. Stock solutions containing the anionic amphiphilic compound, sodium dodecyl sulfate (0.36%), and the preservative, m-cresol (92.3 mM), were prepared by dissolving the solid in water (stocks 2 and 3, respectively). A formulation containing Protein NO: 5 at 10 mg/mL was prepared by addition of an aliquot of stock 1 (4.5 mL) to a 10 mL volumetric flask. Aliquots of stocks 2 (1.0 mL), stock 3 (3.0 mL), and water were added in order. Enough water was added to adjust the final volume to 10 mL. The pH of the solution was checked and, if necessary, the pH was adjusted with µL quantities of dilute HCl or dilute NaOH to a pH 8.5 (± 0.1). The final solution condition was 10 mg/mL of Protein NO: 5, 27.6 mM m-cresol, and 0.036% sodium dodecyl sulfate at pH 8.5 (± 0.1). The solution was then distributed into vials and stored at the desired temperatures.

**Table 1:**

| Formulation stabilization of an obesity protein by representative anionic amphiphilic compounds using a turbidity assay to kinetically monitor aggregation. Each formulation contained 10 mg/mL of Protein No. 5, 27.6 mM m-cresol, and 5 mM arginine at pH 8.5 (± 0.1). Test were performed at 5, 25, and 37°C. Data were collected on days 1, 4, 8, and 14. A routine linear regression analysis was performed on the turbidity data as a function of time. Slopes and intercepts were statistically compared. | | | | |
|---|---|---|---|---|
| Amphiphilic Compound | [compound] (%) | Temp. (°C) | Rate (NTU/day) | y-intercept (NTU) |
| control | 0 | 5 | 0.4‡ , na^{a}(±0.1) | 3.6^{‡ , na} (± 0.5) |
| | 0 | 25 | 5.9^{‡ , na} (±1.1) | 13.9^{*,na} (±9.6) |
| | 0 | 37 | 0.9^{*, na} (±0.34) | 60.9^{‡ , na} (±2.9) |
| dioctyl sodium sulfosuccinate | 0.056 | 5 | 0.1^{†, ‡} (±0.0) | 8.0^{‡ ,‡} (±0.3) |
| | 0.056 | 25 | 0.1^{* , ‡} (±0.1) | 8.5 ^{‡ , *} (±0.4) |
| | 0.056 | 37 | 0.2^{‡ , *} (±0.0) | 10.1^{‡ , ¶} (±0.2) |
| sodium dodecyl sulfate | 0.036 | 5 | 0.8^{†, *} (±0.3) | 6.5^{†, *} (±2.2) |
| | 0.036 | 25 | 0.5^{†, ‡} (±0.1) | 15.7 ^{‡ , *} (±1.1) |
| | 0.036 | 37 | 0.4^{* ,*} (±0.1) | 18.5^{‡ , ‡} (±1.1) |
| sodium laurate | 0.028 | 5 | 0.5^{†, *} (±0.2) | 12.5 ^{‡ , ‡} (±1.3) |
| | 0.028 | 25 | 0.5^{* , ‡} (±0.2) | 21.2^{‡ ,*} (1.6) |
| | 0.028 | 37 | 0.3^{†,*} (±0.1) | 21.5^{‡, ‡} (±0.8) |
| sodium deoxycholate. | 0.049 | 5 | 0.8^{†, *} (±0.1) | 4.1^{†,*} (1.1) |
| | 0.049 | 25 | 2.6^{*,*} (±1.0) | 25.7^{†,*} (±8.5) |
| | 0.049 | 37 | 0.8^{†, *} (±0.2) | 34^{‡ , ‡} (±1.6) |

| | | | | |
|---|---|---|---|---|
| ^{a}The superscript designation, e.g., "*****,*", refers to the statistical evaluation of (1) the probability of the slope being different from zero, "*,*****" and (2) the probability that the slope of the stabilizer formulation is different from the slope of the control formulation, "*****,*". na = not applicable | | | | |
| ^{*}slope or rate, prob. > 0.1 | | | | |
| ^{†}slope or rate, 0.05 < prob. ² 0.1 | | | | |
| ^{‡}slope or rate, 0.001 < prob. ² 0.05 | | | | |
| ^{¶}slope or rate, prob. ² 0.001 | | | | |

### Examples 5-34

**Table II:**

| The ability of an anionic amphiphillic surfacant to decrease the precipitation of a protein of SEQ ID NO: 11 at 10 mg/ml in 10 mM phosphate buffer at pH 7.4 is evaluated as follows: | | | |
|---|---|---|---|
| Experimental Design 10 mM Phosphate Buffer, pH 7.4, 37º | | | |
| Examples | Anionic Amphiphilic Compound | MW | Concentration (Compound to protein ratio) |
| 5, 6 | sodium glycoholate | 487.6 | 2:1, 4:1 |
| 7, 8 | dioctyl sodium sulfosuccinate | 444.57 | 2:1, 4:1 |
| 9, 10 | sodium laurate | 222.3 | 2:1, 4:1 |
| 11, 12 | sodium myristate | 250.4 | 2:1, 4:1 |
| 13, 14 | sodium oleate | 304.4 | 2:1, 4:1 |
| 15, 16 | C-14 GLY Na | 307.37 | 2:1, 4:1 |
| 17, 18 | C-10 PHE Na | 341.42 | 2:1, 4:1 |
| 19, 20 | C-14 PHE Na | 397.52 | 2:1, 4:1 |
| 21, 22 | C-14 norLEU Na | 363.49 | 2:1, 4:1 |
| 23, 24 | C-14 ASP 2Na | 387.4 | 2:1, 4:1 |

| 10 mM Phosphate Buffer, pH 7.4, 25º, 0.3% *m*-cresol | | | |
|---|---|---|---|
| Code | Compound | MW | Concentration |
| 25 | sodium glycoholate | 487.6 | 2:1 |
| 26 | dioctyl sodium sulfosuccinate | 444.57 | 2:1 |
| 27 | sodium laurate | 222.3 | 2:1 |
| 28 | sodium myristate | 250.4 | 2:1 |
| 29 | sodium oleate | 306.5 | 2:1 |
| 30 | C-14 GLY Na | 304.4 | 2:1 |
| 31 | C-10 PHE Na | 341.42 | 2:1 |
| 32 | C-14 PHE Na | 363.49 | 2:1 |
| 33 | C-14 norLEU Na | 363.49 | 2:1 |
| 34 | C-14 ASP 2Na | 387.4 | 2:1 |

All formulations will contain 10 mg/ml SEQ ID NO: 11 in 10 mM phosphate buffer at pH 7.4. All stock solution will be prepared in 10 mM phosphate buffer at pH 7.4. The m-cresol free formulations will be stored at 37º while the m-cresol containing formulations will be stored at 25º. A protein of SEQ ID NO: 11 has a molecular weight of 15967.4; consequently, a 10 mg/ml solution corresponds to 0.627 mM. The time factor will be investigated at 3 levels (Initial, Day 2, Day 4).

### Procedures

Prepare 1 mL of a formulation volumetrically.
1. Prepare all stock solutions.
2. Sterile filter stock B.
3. Add 250 µL of stock solution B to each 2 mL sterile cryogenic vial.
4. Add 750 or 375 µL of appropriate surfactant stock solution.
5. Add 300 µL of m-cresol stock solution if necessary.
6. Bring to 1 ml volume with stock solution A.

| Stock | Molecular Weight | Concentration | Amount |
|---|---|---|---|
| Stock A (pH 7.4, 1L) Na2HPO3 .7H2O | 268.07 | 10 nM | 2.6807 gm |
| Stock B (pH 7.4, 10 mL) Protein | | 40 mg/ml | 430 mg (adjust for purity) |
| Stock A | | | q.s. 10 ml |
| Stock C (pH 7.4, 50 mL) m-cresol | | 1% | 500mg |
| Stock A | | | q.s. 50 ml |
| Stock D-M (pH 7.4, 50 mL) surfactant | | 3.34 mM | check mol. wt. |
| Stock A | | | q.s. 50 ml |

Protein aggregation is monitored by measuring the protein concentration in solution.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

## Claims

1. A soluble parenteral formulation, comprising an obesity protein and an anionic amphiphilic compound.

2. A formulation of Claim 1, wherein the obesity protein is a protein of SEQ ID NO: 15, wherein Xaa is Met.

3. A formulation of Claim 1, wherein the obesity protein is a protein of SEQ ID NO: 15 wherein Xaa is absent.

4. A soluble parenteral formulation, comprising an obesity protein analog and an anionic amphiphilic compound.

5. A formulation of Claim 4, wherein the obesity protein analog is a protein wherein Trp at position 100 is replaced with Ala, Glu, Asp, Gln, or Leu.

6. A formulation of Claim 4 or 5, wherein the obesity protein analog is a protein wherein:
Asn at position 22 is replaced with Gln or Asp;
Thr at position 27 is replaced with Ala;
Met at position 54 is replaced with Leu, or Ala;
Met at position 68 is replaced with Leu;
Asn at position 72 is replaced with Gln or Asp;
Asn at position 78 is replaced with Gln or Asp;
Asn at position 82 is replaced with Gln or Asp; or
Met at position 136 is replaced with Leu, or Ile.

7. A formulation of Claim 4, wherein the obesity protein analog is a protein selected from the group consisting of SEQ ID NO: 2, SEQ ID NO: 3, SEQ ID NO: 5, SEQ ID NO: 6, SEQ ID NO: 7, SEQ ID NO: 8, SEQ ID NO: 9, SEQ ID NO: 10, SEQ ID NO 11, SEQ ID NO: 12, SEQ ID NO: 13, or SEQ ID NO: 14.

8. A formulation of any one of claims 1 through 7, wherein the compound is selected from the group consisting of:
dioctyl sodium sulfosuccinate;
a sulfate salt with an aliphatic chain having from 6 to 20 carbons;
a bile salt;
a salt of a fatty acid;
caprylic acid;
ascorbic acid;
ascorbyl palmitate;
an anionic phospholipid;
an N-acetylated amino acid;
a C₄ to C₂₀ acylated amino acid;
or a combination thereof.

9. A formulation of Claim 8, wherein the compound is selected from the group consisting of:
dioctyl sodium sulfosuccinate;
a sulfate salt with an aliphatic chain having from 6 to 20 carbons;
a bile salt;
a salt of a fatty acid;
caprylic acid;
ascorbic acid;
ascorbyl palmitate;
an anionic phospholipid;
an N-acetylated amino acid;
a C₇ to C₂₀ acylated amino acid;
or a combination thereof.

10. A formulation of Claim 9, wherein the concentration of protein is about 1.0 mg/mL to about 100 mg/mL.

11. A formulation of Claim 10, wherein the concentration of protein is about 5.0 mg/mL to about 50.0 mg/mL.

12. A formulation of Claim 11, which further comprises an isotonicity agent.

13. A formulation of Claim 12, which further comprises a physiologically acceptable buffer.

14. A formulation of Claim 13, which further comprises a pharmaceutically acceptable preservative.

15. A formulation of Claim 14, wherein the preservative is phenol, cresol, or a mixture thereof.

16. A formulation of any one of claims 1 through 15 for use in treating obesity.


SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: Protein Formulations

(iii) NUMBER OF SEQUENCES: 19

(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: 97306420.7
(B) FILING DATE: 22nd August 1997

(2) INFORMATION FOR SEQ ID NO:1:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Modified-site
(B) LOCATION: 28
(D) OTHER INFORMATION: /note= "Xaa at position 28 is Gln
or absent;"

(ix) FEATURE:
(A) NAME/KEY: Modified-site
(B) LOCATION: 4
(D) OTHER INFORMATION: /note= "Gln at position 4 is
replaced with Glu;"

(ix) FEATURE:
(A) NAME/KEY: Modified-site

(B) LOCATION: 7
(D) OTHER INFORMATION: /note= "Gln at position 7 is
replaced with Glu;"

(ix) FEATURE:
(A) NAME/KEY: Modified-site
(B) LOCATION: 22
(D) OTHER INFORMATION: /note= "Asn at position 22 is
replaced with Gln or Asp;"

(ix) FEATURE:
(A) NAME/KEY: Modified-site
(B) LOCATION: 27
(D) OTHER INFORMATION: /note= "Thr at position 27 is
replaced with Ala;"

(ix) FEATURE:
(A) NAME/KEY: Modified-site
(B) LOCATION: 28
(D) OTHER INFORMATION: /note= "Xaa at position 28 is
replaced with Glu;"

(ix) FEATURE:
(A) NAME/KEY: Modified-site
(B) LOCATION: 34
(D) OTHER INFORMATION: /note= "Gln at position 34 is
replaced with Glu;"

(ix) FEATURE:
(A) NAME/KEY: Modified-site
(B) LOCATION: 54
(D) OTHER INFORMATION: /note= "Met at position 54 is
replaced with methionine sulfoxide, Leu, Ile, Val, Ala, or Gly;"

(ix) FEATURE:
(A) NAME/KEY: Modified-site
(B) LOCATION: 56
(D) OTHER INFORMATION: /note= "Gln at position 56 is
replaced with Glu;"

(ix) FEATURE:
(A) NAME/KEY: Modified-site
(B) LOCATION: 62
(D) OTHER INFORMATION: /note= "Gln at position 62 is
replaced with Glu;"

(ix) FEATURE:
(A) NAME/KEY: Modified-site
(B) LOCATION: 63
(D) OTHER INFORMATION: /note= "Gln at position 63 is
replaced with Glu;"

(ix) FEATURE:
(A) NAME/KEY: Modified-site
(B) LOCATION: 68
(D) OTHER INFORMATION: /note= "Met at position 68 is
replaced with methionine sulfoxide, Leu, Ile, Val, Ala, or Gly;"

(ix) FEATURE:
(A) NAME/KEY: Modified-site
(B) LOCATION: 72

(D) OTHER INFORMATION: /note= "Asn at position 72 is
replaced with Gln, Glu, or Asp;"

(ix) FEATURE:
(A) NAME/KEY: Modified-site
(B) LOCATION: 75
(D) OTHER INFORMATION: /note= "Gln at position 75 is
replaced with Glu;"

(ix) FEATURE:
(A) NAME/KEY: Modified-site
(B) LOCATION: 77
(D) OTHER INFORMATION: /note= "Ser at position 77 is
replaced with Ala;"

(ix) FEATURE:
(A) NAME/KEY: Modified-site
(B) LOCATION: 78
(D) OTHER INFORMATION: /note= "Asn at position 78 is
replaced with Gln or Asp;"

(ix) FEATURE:
(A) NAME/KEY: Modified-site
(B) LOCATION: 82
(D) OTHER INFORMATION: /note= "Asn at position 82 is
replaced with Gln or Asp;"

(ix) FEATURE:
(A) NAME/KEY: Modified-site
(B) LOCATION: 97
(D) OTHER INFORMATION: /note= "His at position 97 is
replaced with Gln, Asn, Ala, Gly, Ser, or Pro;"

(ix) FEATURE:
(A) NAME/KEY: Modified-site
(B) LOCATION: 100
(D) OTHER INFORMATION: /note= "Trp at position 100 is
replaced with Ala, Glu, Asp, Asn, Met, Ile, Phe, Tyr, Ser, Thr,
Gly, Gln, Val or Leu;"

(ix) FEATURE:
(A) NAME/KEY: Modified-site
(B) LOCATION: 101
(D) OTHER INFORMATION: /note= "Ala at position 101 is
replaced with Ser, Asn, Gly, His, Pro, Thr, or Val;"

(ix) FEATURE:
(A) NAME/KEY: Modified-site
(B) LOCATION: 102
(D) OTHER INFORMATION: /note= "Ser at position 102 is
replaced with Arg;"

(ix) FEATURE:
(A) NAME/KEY: Modified-site
(B) LOCATION: 103
(D) OTHER INFORMATION: /note= "Gly at position 103 is
replaced with Ala;"

(ix) FEATURE:
(A) NAME/KEY: Modified-site
(B) LOCATION: 105

(D) OTHER INFORMATION: /note= "Glu at position 105 is
replaced with Gln;"

(ix) FEATURE:
(A) NAME/KEY: Modified-site
(B) LOCATION: 106
(D) OTHER INFORMATION: /note= "Thr at position 106 is
replaced with Lys or Ser;"

(ix) FEATURE:
(A) NAME/KEY: Modified-site
(B) LOCATION: 107
(D) OTHER INFORMATION: /note= "Leu at position 107 is
replaced with Pro;"

(ix) FEATURE:
(A) NAME/KEY: Modified-site
(B) LOCATION: 108
(D) OTHER INFORMATION: /note= "Asp at position 108 is
replaced with Glu;"

(ix) FEATURE:
(A) NAME/KEY: Modified-site
(B) LOCATION: 111
(D) OTHER INFORMATION: /note= "Gly at position 111 is
replaced with Asp;"

(ix) FEATURE:
(A) NAME/KEY: Modified-site
(B) LOCATION: 118
(D) OTHER INFORMATION: /note= "Gly at position 118 is
replaced with Leu;"

(ix) FEATURE:
(A) NAME/KEY: Modified-site
(B) LOCATION: 130
(D) OTHER INFORMATION: /note= "Gln at position 130 is
replaced with Glu;"

(ix) FEATURE:
(A) NAME/KEY: Modified-site
(B) LOCATION: 134
(D) OTHER INFORMATION: /note= "Gln at position 134 is
replaced with Glu;"

(ix) FEATURE:
(A) NAME/KEY: Modified-site
(B) LOCATION: 136
(D) OTHER INFORMATION: /note= "Met at position 136 is
replaced with methionine sulfoxide, Leu, Ile, Val, Ala, or Gly;"

(ix) FEATURE:
(A) NAME/KEY: Modified-site
(B) LOCATION: 138
(D) OTHER INFORMATION: /note= "Trp at position 138 is
replaced with Ala, Glu, Asp, Asn, Met, Ile, Phe, Tyr, Ser, Thr,
Gly, Gln, Val or Leu;"

(ix) FEATURE:
(A) NAME/KEY: Modified-site
(B) LOCATION: 139

(D) OTHER INFORMATION: /note= "Gln at position 139 is
replaced with Glu; or a pharmaceutically acceptable salt
thereof."

(xi) SEQUENCE DESCRIPTION: SEQ ID NO:1:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Xaa Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:2:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:2:

Val Pro Ile Gln Lys Val Gln Asp Asp Thr Lys Thr Leu Ile Lys Thr
1               5                   10                  15

Ile Val Thr Arg Ile Asp Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:3:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:3:

Val Pro Ile Gln Lys Val Gln Asp Asp Thr Lys Thr Leu Ile Lys Thr
1               5                   10                  15

Ile Val Thr Arg Ile Asn Asp Ile Ser His Ala Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro

130                 135                 140

Gly Cys
145

(2) INFORMATION FOR SEQ ID NO:4:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Modified-site
(B) LOCATION: 97
(D) OTHER INFORMATION: /note= "His at position 97 is
replaced with Gln, Asn, Ala, Gly, Ser, or Pro;"

(ix) FEATURE:
(A) NAME/KEY: Modified-site
(B) LOCATION: 100
(D) OTHER INFORMATION: /note= "Trp at position 100 is
replaced with Ala, Glu, Asp, Asn, Met, Ile, Phe, Tyr, Ser, Thr,
Gly, Gln, Val or Leu;"

(ix) FEATURE:
(A) NAME/KEY: Modified-site
(B) LOCATION: 101
(D) OTHER INFORMATION: /note= "Ala at position 101 is
replaced with Ser, Asn, Gly, His, Pro, Thr or Val;"

(ix) FEATURE:
(A) NAME/KEY: Modified-site
(B) LOCATION: 102
(D) OTHER INFORMATION: /note= "Ser at position 102 is
replaced with Arg;"

(ix) FEATURE:
(A) NAME/KEY: Modified-site
(B) LOCATION: 103
(D) OTHER INFORMATION: /note= "Gly at position 103 is
replaced with Ala;"

(ix) FEATURE:
(A) NAME/KEY: Modified-site
(B) LOCATION: 105
(D) OTHER INFORMATION: /note= "Glu at position 105 is
replaced with Gln;"

(ix) FEATURE:
(A) NAME/KEY: Modified-site
(B) LOCATION: 106
(D) OTHER INFORMATION: /note= "Thr at position 106 is
replaced with Lys or Ser;"

(ix) FEATURE:
(A) NAME/KEY: Modified-site

(B) LOCATION: 107
(D) OTHER INFORMATION: /note= "Leu at position 107 is
replaced with Pro;"

(ix) FEATURE:
(A) NAME/KEY: Modified-site
(B) LOCATION: 108
(D) OTHER INFORMATION: /note= "Asp at position 108 is
replaced with Glu;"

(ix) FEATURE:
(A) NAME/KEY: Modified-site
(B) LOCATION: 111
(D) OTHER INFORMATION: /note= "Gly at position 111 is
replaced with Asp;"

(ix) FEATURE:
(A) NAME/KEY: Modified-site
(B) LOCATION: 138
(D) OTHER INFORMATION: /note= "Trp at position 138 is
replaced with Ala, Glu, Asp, Asn, Met, Ile, Phe, Tyr, Ser, Thr,
Gly, Gln, Val or Leu; or a pharmaceutically acceptable salt
thereof."

(xi) SEQUENCE DESCRIPTION: SEQ ID NO:4:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:5:

(i) SEQUENCE CHARACTERISTICS:
(A) LENGTH: 146 amino acids

(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:5:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:6:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:6:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:7:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:7:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:8:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:8:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:9:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:9:

Val Pro Ile Gln Lys Val Gln Asp Asp Thr Lys Thr Leu Ile Lys Thr
1               5                   10                  15

Ile Val Thr Arg Ile Asn Asp Ile Ser His Ala Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:10:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:10:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:11:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:11:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Glu Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:12:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:12:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Asp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:13:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

(xi) SEQUENCE DESCRIPTION: SEQ ID NO:13:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Gln Thr Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:14:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:14:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:15:

     (i) SEQUENCE CHARACTERISTICS:
          (A) LENGTH: 147 amino acids
          (B) TYPE: amino acid
          (C) STRANDEDNESS: single
          (D) TOPOLOGY: linear

    (ii) MOLECULE TYPE: protein


    (ix) FEATURE:
          (A) NAME/KEY: Modified-site
          (B) LOCATION: 1
          (D) OTHER INFORMATION: /note= "Xaa at position 1 is Met or
  absent"


    (xi) SEQUENCE DESCRIPTION: SEQ ID NO:15:

Xaa Val Pro Ile Gln Lys Val Gln Asp Asp Thr Lys Thr Leu Ile Lys
1               5                   10                  15

Thr Ile Val Thr Arg Ile Asn Asp Ile Ser His Thr Gln Ser Val Ser
            20                  25                  30

Ser Lys Gln Lys Val Thr Gly Leu Asp Phe Ile Pro Gly Leu His Pro
        35                  40                  45

Ile Leu Thr Leu Ser Lys Met Asp Gln Thr Leu Ala Val Tyr Gln Gln
    50                  55                  60

Ile Leu Thr Ser Met Pro Ser Arg Asn Val Ile Gln Ile Ser Asn Asp
65                  70                  75                  80

Leu Glu Asn Leu Arg Asp Leu Leu His Val Leu Ala Phe Ser Lys Ser
                85                  90                  95

Cys His Leu Pro Trp Ala Ser Gly Leu Glu Thr Leu Asp Ser Leu Gly
            100                 105                 110

Gly Val Leu Glu Ala Ser Gly Tyr Ser Thr Glu Val Val Ala Leu Ser

115                 120                 125

Arg Leu Gln Gly Ser Leu Gln Asp Met Leu Trp Gln Leu Asp Leu Ser
    130                 135                 140

Pro Gly Cys
145

(2) INFORMATION FOR SEQ ID NO:16:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:16:

ATGCATTGGG GAMCCTGTG
19

(2) INFORMATION FOR SEQ ID NO:17:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:17:

GGATTCTTGT GGCTTTGGYC TATCT
25

(2) INFORMATION FOR SEQ ID NO:18:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:18:

TCAGCACCCA GGGCTGAGGT CCA
23

(2) INFORMATION FOR SEQ ID NO:19:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:19:

CATGTCCTGC AGAGACCCCT GCAGCCTGCT CA
32